# EUROPEAN PATENT APPLICATION

(11) **EP 4 151 188 A1**
(43) Date of publication of application: **22.03.2023**
(21) Application number: 22151371.6
(22) Date of filing: 13.01.2022
(51) Int. Cl.: A61F 11/00

(54) **TOOL FOR REMOVING EARWAX FROM THE EAR CANAL**

(30) Priority: 20.09.2021 ES 202131858 U
(71) Applicant: Ibernova Aragón, S.L., 50009 Zaragoza (ES)
(72) Inventor: MIHALUT GHERGUT, Gabriel, 50009 Zaragoza (ES)
(74) Representative: Ungria López, Javier

(57) **Abstract**

The present invention relates to a funnel-shaped tool for removing earwax from the ear canal that is made up of a plant tissue comprising between 70% to 100% hemp fibres, and wherein said plant tissue has a wax coating comprising at least 90% beeswax. The present invention is also related to the method for obtaining the tool object of the invention. Finally, the present invention relates to the use of the tool object of the invention for removing earwax from the ear canal by means of the combustion of said tool.

## Description

### OBJECT OF THE INVENTION

The present invention relates to a tool for removing earwax from the ear canal, and preferably for removing an earwax plug from the ear canal. This invention falls within the audiology industry, and the industry that develops personal hygiene products and devices.

### BACKGROUND OF THE INVENTION AND TECHNICAL PROBLEM TO SOLVE

Earwax is a natural secretion produced by our body that serves as a barrier to prevent bacteria from entering inside. However, when this secretion is excessive, it can generate what is known as an ear or earwax plug, which can be uncomfortable or even very painful, so it is necessary to develop a method or products for the removal thereof.

The two most common ways in the state of the art for removing earwax are:
- Pressurised warm water is irrigated through a syringe, with the aim of expelling the plug through the ear canal. This manoeuvre is dangerous, as it can lead to serious middle ear infections. It is also not recommended in the event of oedema in the external ear canal, having had any surgery on the ear, vertigo, cholesteatoma, etc.
- The removal of the plug is performed by means of tweezers and an aspirator, but this manoeuvre is quite complex and should only be done by an otolaryngologist.

For this reason, the audiology industry has seen the need to develop different tools that enable the earwax plug to be removed from the ear canal, avoiding the possible risks of the techniques indicated above. Currently, there are devices of different types, either in the configuration or material, and shape thereof, as well as compositions capable of removing an earwax plug from the ear canal. Here are some relevant examples for removing earwax plugs from the ear canal:
- International application WO2012158382, entitled "Device and method for removing earwax" describes an earwax removal device having varying structures to provide different earwax removal capabilities. In all designs, the overall contour of the earwax-extracting piece resembles the dimensions of the external ear canal and is flexible enough to conform to it during insertion. Embodiments include a club-like piece having multiple protrusions and extractions to collect wax along its sides, and an opening in the tip that leads to a channel to collect wax; a flexible spiral member to compress when the device is inserted in an ear and thereby extract wax as it expands when the device is removed; a screw-like structure to laterally move wax and grind as it rotates; a multi-channel structure to cut and collect wax simultaneously; and a shovel-like structure to effectively shear and scrape wax from an ear canal.
- International application WO2009063978, entitled "Earwax removing tool" which describes a tool for removing wax that comprises a rodlike shaft portion, and a protrusion formed along the periphery of the shaft portion wherein the protrusion can be inserted into the external auditory miatus. The protrusion has a plurality of holders composed of an elastic material and supported on the shaft portion and earwax can be held between holders adjoining along the formation direction of the protrusion.
- International application 2010140144 entitled "Ear cleaning device" describes a device comprising a handle, a neck portion and a spoon-like cleaning head which has an overall flexibility that would be stiff enough to enable efficient earwax extraction, but would inwardly bend to prevent ear trauma under an applied pressure which is high enough to injure the ear canal skin. The neck portion also has the ability to bend in the event that the pressure against the canal wall continues. Another cleaning head may be added to the opposite end which may be identical to the said cleaning head, made of an absorbent material to absorb secretions, or serve as an aid stick for personal hygiene.
- Japanese application JP1113781 entitled "Ear care tool" describes an ear care tool comprising a first combustion portion formed by a sheet of natural fibre impregnated with wax or beeswax and perfume in the form of a hollow conical cylinder and a second cylindrical insert portion made of a soft and heat resistant plastic material. Said portions form a single body and are attached by means of a ring-shaped groove wherein a ring-shaped fireproof protection tray is arranged.

However, these devices are quite complex and require, based on the configuration thereof, that a person having knowledge and practice about its use, can use it, making it very difficult for an end user to acquire it for their own use and personal hygiene.

In addition, none of these devices are utensils that are easy to use, ecological, recyclable and have a double action of cleaning and disinfecting the ear canal.

That is why there is a need to develop a tool that represents an alternative to the devices and tools that are already known in the state of the art, which is made of natural materials and therefore is a sustainable and ecological tool, easy to use and that has a double treatment effect for removing said earwax and antimicrobial effect.

### DESCRIPTION OF THE INVENTION

As explained above, the inventors propose an alternative tool to what is already known in the state of the art capable of removing an earwax plug from the ear canal in a safe and non-traumatic way for the user. In addition, this tool stands out because due to the composition of the materials forming it, firstly, they have antimicrobial and anti-inflammatory properties, but it is also recyclable and ecological.

In order to achieve the objectives and avoid the drawbacks mentioned in the previous sections, the invention proposes a tool having a funnel or hollow cone shape and that is made of a tissue of plant origin comprising between 70% to 100% hemp and a coating made of beeswax.

Therefore, in a first aspect, the invention relates to a tool (1) for removing an earwax plug from the ear canal, characterised in that:
- it has a hollow cone shape and a lateral length from the vertex (1.1) to the base (1.2) thereof that is comprised between 16 to 25 cm, and
- it is made up of a plant tissue comprising between 70% to 100% hemp and which in turn has a natural wax coating comprising at least 90% beeswax.

In the context of the present invention, a hollow cone is understood as a geometric body formed by a curved and closed lateral surface, which ends in a first narrow end which is called the vertex, and a second opposite end which is a plane with a circular shape which is called the base and wherein said cone is empty inside, exhibiting a hole with a different diameter at each of the ends thereof.

Likewise, in the context of the present invention, the distance or length between the vertex and the base is called lateral length. This length is also called the generatrix because of the geometric shape of the cone. In particular embodiments of the present invention, the lateral length or generatrix is comprised between 16 cm and 25 cm, preferably being between 18 cm and 24 cm, and more preferably, between 21 cm and 23 cm. In particular embodiments of the present invention, the lateral length can be 16 cm, 17 cm, 18 cm, 19 cm, 20 cm, 21 cm, 22 cm, 23 cm, 24 cm or 25 cm.

Taking into account that the tool object of the invention is a hollow cone as indicated above, in particular embodiments its vertex has a hole with a diameter that can be between 4 mm and 10 mm, preferably being between 5 mm and 8 mm. In particular embodiments of the present invention, the diameter of the vertex hole can be 4 mm, 5 mm, 6 mm, 7 mm, 8 mm, 9 mm or 10 mm.

In particular embodiments, taking into account that the tool object of the invention is a hollow cone, its base has a hole with a diameter that can be comprised between 2 cm and 3.5 cm, preferably being between 2.5 cm and 3 cm. In particular embodiments of the present invention, the diameter of the hole in the base can be 2 cm, 2.5 cm, 3 cm or 3.5 cm.

In particular embodiments of the present invention, the wall of the tool object of the invention is made up of a natural tissue, particularly being a plant tissue. Based on this, plant tissue is understood as a tissue that is 100% made up of fibres of plant origin wherein said plant fibres are obtained from plants or trees, which are braided or interwoven.

In particular embodiments of the present invention, the plant tissue comprises between 70% and 100% hemp fibres. In preferred embodiments of the present invention, the natural tissue comprises between 75% and 97% hemp, even more preferably being between 85% and 95% hemp. In other particular embodiments of the present invention, the natural tissue can comprise 70%, 75%, 80%, 85% or 90% of hemp fibres. In other particular embodiments of the present invention, the plant tissue can comprise 91%, 92%, 93%, 94%, 95%, 96%, 97%, 98%, 99% or 100% hemp fibres. It should be noted that hemp is an ecological material of plant origin. Hemp fibres are obtained from hemp seeds that are rich in plant protein that contributes to tissue regeneration and also comprises a higher proportion of essential fatty acids compared to the seeds of any other plant, which contribute to the immune response from which the surprising antifungal, antibacterial and antimicrobial properties thereof are derived.

In other preferred embodiments herein, the plant tissue comprises up to a maximum of 30% plant fibres that are selected from the group consisting of cotton, reed, palm, esparto, loofah, linen, jute fibres and a combination of the above, preferably being cotton, palm or jute fibres. In the context of the present invention, these fibres listed above are also called plant fibre-additive. In preferred embodiments, the plant tissue comprises between 1% and 30% plant fibre-additive, preferably between 2% and 10%, and even more preferably, between 2% and 5% plant fibre-additive. In other particular embodiments of the present invention, the plant tissue can comprise 1%, 2%, 3%, 4%, 5%, 6%, 7%, 8%, 9% or 10%.

In other particular embodiments of the tool object of the invention, the natural tissue comprises a natural wax coating. Wax is a solid, white or yellow substance, meltable and insoluble in water, of animal, plant or mineral origin, which is very malleable and has various uses. In the context of the present invention, the natural wax coating is a coating that is made up of 100% animal wax or vegetable wax, without any artificial or synthetic additives.

In a particular embodiment of the present invention, the coating of the present invention comprises at least 90% beeswax, preferably 90% pure beeswax. In preferred embodiments of the present invention, the natural wax coating comprises beeswax between 90% and 100%, preferably between 95% and 99 %. In other particular embodiments, the amount of beeswax that the coating comprises can be 90%, 91%, 92%, 93%, 94%, 95%, 96%, 97%, 98%, 99% or 100%.

In the context of the present invention, pure beeswax is understood to be a natural product made by bees, and having a composition of 99.5%, 99.75%, 99.95% or 100% beeswax.

In other preferred embodiments of the present invention, the natural wax coating may comprise an additive which is selected from the group consisting of carnauba wax, jojoba wax, or a combination of the two. In the context of the present invention, the aforementioned waxes are also called a wax-additive. In preferred embodiments of the present invention, the coating can comprise at least one wax-additive and can be comprised between 1% to 10%, preferably between 2% and 8%, and even more preferably, between 2% and 5%. In other particular embodiments, the amount of wax-additive that the coating comprises can be 1%, 2%, 3%, 4%, 5%, 6%, 7%, 8%, 9% or 10%.

In a preferred embodiment of the present invention, the tool (1) has a ring (2) that is perpendicular to the longitudinal axis of the tool and concentric with the holes of the vertex and of the base (1) that is at least one quarter of the total lateral length of the tool away from the vertex (1.1). In the context of the present invention, this ring (2), which is also called the extinguishing ring, has the function of determining at what distance from the vertex (1.1) the combustion of the tool (1) must be stopped in order to be safe for the user. In another preferred embodiment of the present invention, the extinguishing ring is at 5 cm, 6 cm, 7 cm or 8 cm.

In preferred embodiments of the present invention, the ring (2) is a mark of a colour different from the colour of the wall of the tool (1), which, preferably, can be red or black. It should be noted that the colour is irrelevant as long as the colour of the ring (2) stands out with respect to the colour of the wall of the tool (1).

In other preferred embodiments of the present invention, the ring (2) can be made of a self-extinguishing material. In the context of the present invention, a self-extinguishing material is defined as a material that has the ability to stop burning once the heat source causing the flame has been removed. In more preferred embodiments, the ring is made of cork. Cork is a difficult-to-burn and even fireproof material. Cork agglomerate is made up of cork grains, bound together by the natural cork resin itself, which is suberin, by means of a cooking process. Thanks to this preparation, the cork improves its thermal behaviour, making it fireproof. In addition, it withstands temperatures of up to 120 °C and does not release toxic fumes when burnt, which is a great advantage over other materials. In addition, as cork is obtained from renewable raw material, without the use of synthetic agents, as well as the ability to be recycled for other purposes, it makes this extinguishing ring an ecological and environmentally friendly product.

In particular embodiments of the present invention, the extinguishing ring that the tool object of the invention may comprise may have a thickness comprised between 5 to 10 mm, preferably between 8 mm to 10 mm. In particular embodiments of the present invention, the thickness of the extinguishing ring can be 5 mm, 6 mm, 7 mm, 8 mm, 9 mm or 10 mm.

To manufacture this device, the starting point is a strip of plant tissue comprising 70% to 100% hemp fibres and being 1.5 to 6 cm wide, preferably between 3 to 4 cm and from 15 cm to 60 cm long.

In particular embodiments, the width of the strip of plant tissue is between 1.5 cm to 6 cm, and preferably between 3 cm to 5 cm. In particular embodiments of the present invention, the width of the strip of plant tissue can be 1.5 cm, 2 cm, 2.5 cm, 3 cm, 3.5 cm, 4 cm, 4.5 cm, 5 cm, 5.5 cm or 6 cm.

In particular embodiments, the length of the strip of plant tissue is comprised between 15 cm to 60 cm, and preferably between 30 cm to 50 cm. In particular embodiments of the present invention, the length of the strip of plant tissue can be 15 cm, 20 cm, 25 cm, 30 cm, 35 cm, 40 cm, 45 cm, 50 cm, 55 cm or 60 cm.

Said strip a natural wax coating comprising at least 90% melted pure beeswax that is at a temperature comprised between 62 °C to 65 °C. Preferably, said beeswax film has a thickness of 1 to 5 mm, and preferably 2 mm.

Next, the strip of plant tissue is soaked in the wax coating as defined herein. The wax coating is heated to a temperature comprised between 75 °C to 90 °C to bring it to a liquid phase. In this way, once the wax is brought to a liquid phase, the strip of plant tissue is soaked in said liquid wax. In the context of the present invention, it is understood that the strip is soaked in said liquid wax until it admits no more wax on the surface thereof.

Subsequently, said strip of plant tissue is left to cool to a temperature comprised between 50 °C to 65 °C, such that the wax coating exhibits a viscous texture, but has not solidified yet, such that it is used as a natural adhesive for attaching the plant strip to itself and shape the tool (1).

To shape the tool (1) in the shape of a hollow cone, it is wound around a cone-shaped mould following a helical movement, from the vertex of the mould to the other end of the mould, which is the circular base, winding said strip about the mould until obtaining a hollow cone with a lateral length between 16 to 25 cm.

During winding, half the width of strip that is being placed on the mould overlaps the half width of strip that is already arranged on the mould in such a way that the wax coating acts as a bonding product. In this way, the tool object of the invention is obtained without adding any type of artificial bonding compound, obtaining a product made of natural and ecological materials. Subsequently, it was left to cool to room temperature.

In a particular embodiment of the present invention, one face of the strip is soaked with the beeswax coating described herein. In this way, one face of the strip is soaked with the coating and the other face of the strip is not coated. In this preferred embodiment, during winding, the face of the strip that is soaked with the coating is arranged towards the surface of the mould. For this reason, in this preferred embodiment, half the width of the strip that is being placed on the mould overlaps the half width of strip that is already arranged on the mould, wherein the face of the strip that is arranged towards the mould is the face that is soaked in the coating, and in such a way that said wax coating acts as a bonding product.

In the context of the present invention, ambient temperature is understood to be a temperature comprised between 18 °C and 25 °C, preferably between 20 °C and 25 °C, more preferably between 22 °C and 23 °C, even more preferably 23 °C.

In particular embodiments of the present invention, when the tool object of the invention comprises an extinguishing ring (2), the ring is a mark of a colour different from the colour of the wall of the tool (1) or it can be made of a self-extinguishing material. It should be noted that the extinguishing ring is the one described earlier herein. Based on this, in a preferred embodiment of the present invention, once the tool has been formed, a mark is made of a colour different from the colour of the wall of the tool and with a thickness comprised between 5 and 10 mm, preferably red in colour, at least one quarter of the total lateral length of the tool away from the vertex.

In these preferred embodiments of the present invention, the colour marking is made immediately after the tool has been shaped on the mould or after the tool has cooled down to room temperature, comprised between 18 °C and 25 °C.

In particular embodiments of the present invention, when the tool object of the invention comprises an extinguishing ring (2), wherein the ring is made of a self-extinguishing material, the extinguishing ring is bonded immediately after the tool has been shaped on the mould, since the coating is still in the semiliquid phase due to the fact that the tool is at a temperature comprised between 50 °C to 65 °C. In this way, the coating itself acts as a bonder so that the material from which the extinguishing ring is made bonds to the wall of the tool. In these particular embodiments, once the ring of self-extinguishing material has bonded, the tool is left to cool to a room temperature comprised between 18 °C and 25 °C.

Finally, the tool is demoulded and left to settle in a place or receptacle with little light, dry and cool for at least 10 days before use, preferably between 10 to 15 days. In particular embodiments of the present invention, it is left to settle for 10, 11, 12, 13, 14 or 15 days.

In the context of the present invention it is indicated that the tool object of the invention is left to settle in a cool place, understood as a place or receptacle at a temperature comprised between 18 °C and 23 °C, preferably between 18 °C and 20 °C.

In another aspect of the present invention, the tool according to the first aspect of the present invention can be used for removing earwax in the ear canal by means of the combustion of said tool (1) and, preferably, for removing an earwax plug from the ear canal. In this way, to remove earwax, the vertex of the tool is placed at the outlet on the outside of the ear canal, placing the tool in the same direction as the longitudinal axis of the ear canal, or what is the same, perpendicular to the surface of the auditory chamber and is set on fire at the opposite end. In particular embodiments of the present invention, the tool (1) object of the invention is used for removing earwax from the ear canal by means of the combustion of said tool. Preferably, the tool (1) object of the invention is used for removing an earwax plug from the ear canal by means of the combustion of said tool.

Preferably, the combustion of the tool (1) lasts for a time comprised between 8 to 15 minutes, more preferably between 10 and 12 minutes. In particular embodiments, the combustion can last 8, 9, 10, 11, 12, 13, 14 or 15 minutes.

Due to the shape of the tool (1), the heat generated by the combustion of the tool (1) is directed towards the external ear canal, causing the earwax plug to dissolve gradually and smoothly. That is why the tool is used for gently removing an earwax plug, without being traumatic for its user.

It should be noted that due to the nature of the plant tissue and the coating thereof, combustion is slow and completely safe for the user.

The heat that is transmitted through the inside of the tool towards the ear canal has two direct actions on the earwax plug, causing it to soften due to the temperature generated in the ear canal as a consequence of the heat generated inside the tool, and simultaneously, a chimney effect is generated between the vertex of the funnel and the area comprised between the tool (1) and the ear canal, generating a vacuum that safely, in a relaxing and non-invasive manner, extracts the excess earwax from the ear canal. Indirectly, this slight vacuum also helps to balance the internal auricular pressure, removing possible discomfort derived from the earwax plug such as buzzing, a feeling of blocked ears, and even dizziness and migraines.

It should be noted that due to the material from which the tool object of the invention is made, and in particular, to the high percentage of hemp fibres and the wax coating, when the tool (1) is burnt, the smoke resulting from the combustion has a relaxing, disinfectant and anti-inflammatory action on the area of the external ear canal where the earwax is located, contributing to remove it and alleviating the discomfort and pain possibly caused by excess earwax, preferably an earwax plug, and even removing the presence of some microorganism that may have accumulated in the ear canal due to its drainage being limited by the excessive presence of earwax.

Likewise, it should be noted that the scent released by the tool (1) at the time of combustion is strong, penetrating, sweet and preferably smells like honey.

Due to the fact that the tool (1) is made up of a natural tissue comprising between 70% and 100% hemp fibres and a natural wax coating comprising at least 90% beeswax and the configuration and dimensions of said tool (1) described herein, the combustion of said tool lasts for a time comprised between 8 to 15 minutes, preferably between 10 to 15 minutes and, even more preferably between 12 and 15 minutes. In particular embodiments, the burning time of the tool can be 8, 9, 10, 11, 12, 13, 14 or 15 minutes.

The combustion of the tool can be comprised during the period of time indicated above, since the presence of the natural wax coating of the plant tissue causes the combustion to be slow, allowing the heat to act on the ear canal effectively removing the earwax.

In order to effectively remove earwax from the ear canal, and preferably an earwax plug from the ear canal, the combustion of the tool (1) according to the first aspect of the invention is performed once per day. In particular embodiments, the combustion of the tool (1) can be performed up to a maximum of 3 days in a row and once per day. In preferred embodiments of the present invention, the combustion of the tool is performed one day or two days in a row and once per day or for 3 days in a row and once per day.

Likewise, the presence of the natural wax coating of the plant tissue has another effect and that is that during the combustion of the tool no ashes are generated that can fall into the ear canal, making this tool a very safe instrument for the user, and also practical, since there is no need to use an additional utensil to prevent possible ashes from falling on the user.

Due to the composition of the materials making it up and the configuration thereof, the tool (1) object of the invention has numerous advantages, among which the following are to be highlighted:
- It is an economical product due to the nature of the materials from which the device object of the invention is made;
- It is a disposable product;
- Compared with other devices of the state of the art, as it is made of natural materials, it is sustainable, so that the waste derived from both the manufacture and the use thereof does not cause pollution;
- Compared with other devices that are made of metal or plastics, due to the very nature of the materials making it up, the repetitive use thereof can be irritating to the external ear, so its frequent use can have risks. On the contrary, the device described herein can be used as many times as necessary;
- Due to the high proportion of hemp in the material from which the device is made, the device described herein has antimicrobial, and in particular, antifungal and antimicrobial features;
- This system enables the earwax to be removed from the ear canal in a non-traumatic manner.
- Another advantage derived from the materials that the device object of the invention is made of, and in particular, from the combination of a material made from plant fibres and preferably from 60% to 100% hemp combined with beeswax, is that it prevents ashes that can fall into the ear canal from forming, making it unnecessary to use other complementary parts that must be added to the device for use thereof.

All the terms and embodiments described above are applicable to any aspect and embodiment of the invention. According to the present invention, the singular term "the", "a", "one", is also related to the corresponding plural thereof unless it is clear from the context that the term clearly relates to a species in the singular. The term "comprises" or "comprising", as used herein, also describes "consists of" or "consisting of" in accordance with generally accepted patent practice.

### BRIEF DESCRIPTION OF THE FIGURES

To complete the description of the invention, and for the purpose of helping to make the features thereof more readily understandable, according to a preferred exemplary embodiment thereof, a set of drawings is included wherein, by way of illustration and not limitation, the following figure has been represented:

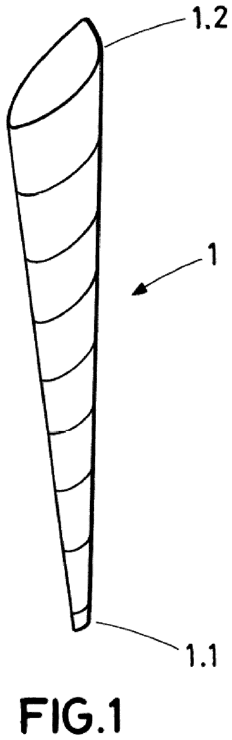

- Figure 1. Side view of a prototype of the tool object of the invention.

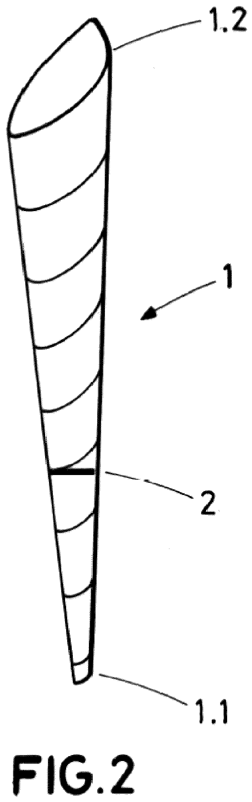

- Figure 2. Side view of a prototype of the tool object of the invention comprising the extinguishing ring.
- Figure 3. Fig. 3.A. Representation of the beginning of the use of the tool object of the invention for removing the earwax plug. In this representation, the earwax is observed inside the ear and how the tool begins to burn. Fig. 3.B. A representation of the final moment of the use of the tool wherein the tool has been consumed by fire almost two thirds of its lateral length and it is also observed that the earwax plug has already been removed.
- Figure 4. It is a representation of the final moment of the use of the tool wherein the tool has been consumed by fire almost two thirds of its lateral length and it is also observed that the earwax plug has already been removed.

A list of the references used in the figures is provided below:
1. Representation of the tool
1.1 Representation of the vertex of the tool
1.2 Representation of the base of the tool
2. Representation of the extinguishing ring.

### DESCRIPTION OF A PREFERRED EMBODIMENT OF THE INVENTION

The following invention is described by means of the following examples, which are to be interpreted as merely illustrative and not limiting of the scope of the invention.

### Description of 3 tested prototypes

A preferred embodiment of the present invention is a tool having a hollow cone shape with a lateral length that is 20 cm long, the apex of which has a diameter of 6 mm and the base of which has a diameter of 3 cm.

Three prototypes of tools were made with the dimensions indicated above, and which were made of 3 different plant materials:
1. Plant tissue 1, comprising:
   a. 90% hemp fibres, and
   b. 10% esparto fibres
2. Plant tissue 2, comprising:
   a. 95% hemp fibres, and
   b. 5% reed fibres
3. Plant tissue 3 comprising 100% hemp fibres

### Obtaining the 3 prototypes of the tool

For the above 3 plant tissues, a strip of the plant tissue having a width of 5 cm and a length of 50 cm was prepared by cutting.

Subsequently, the strips obtained in each of the cases were soaked in a coating comprising 99% pure beeswax and 1% jojoba wax that was at a temperature of 85 °C.

Once the strips were soaked with the coating indicated above, they were left to cool to a temperature of 50 °C and, then, they were arranged on a cone-shaped mould from the first end, which is the vertex, to the opposite end, which is the base, winding the strip of plant tissue around the mould following a helical movement until it forms the 3 cones with the same dimensions indicated above.

### Adding the extinguishing ring

It is worth noting that prototype 1, made from plant tissue 1, did not have an extinguishing ring.

In the case of prototype 2, made from plant tissue 2, a 5 mm thick mark red in colour was placed at a 6 cm distance from the vertex of the prototype as an extinguishing ring.

Furthermore, prototype 3, made from plant tissue 3, had an extinguishing ring that was a 6 mm thick cork ring, which was bonded to the wall of the tool due to the coating at a 5 cm distance from the vertex of the prototype.

Finally, they were left to cool to a room temperature of 23 °C and, subsequently, the three prototypes were left to settle for 15 days, at a temperature of 18 °C.

### Use of the 3 prototypes prepared for removing an earwax plug

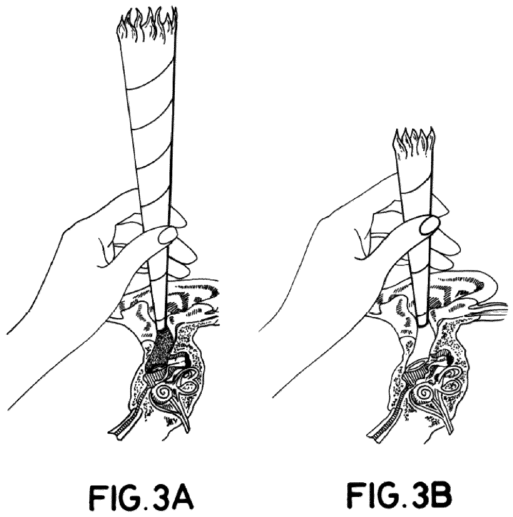

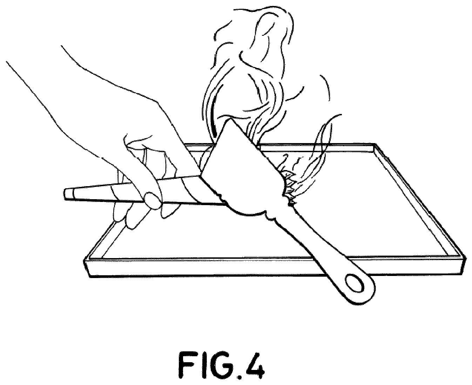

To remove an earwax plug, prototype 1 was placed in the same direction of the longitudinal axis of the ear canal, or what is the same, perpendicular to the surface of the auditory chamber and set on fire at the opposite end, as shown in Figure 3A and 3B. Once the flame approached one quarter of the total lateral length of the tool away from the vertex, the tool was removed from the external ear canal, and the flame was extinguished. To extinguish the flame, the funnel was placed on a tray and a clean cut was made over the area that was burning, as shown in Figure 4.

In the case of prototype 2, it was used in the same way as in the case of prototype 1, with the difference that said tool included a mark at a 6 cm distance from the vertex. Therefore, when the flame reached said mark, the tool was removed from the ear canal, and the flame was extinguished in the same way as in the case of prototype 1.

In the case of prototype 3, it was used in the same way as in the two previous cases 1 and 2 with the difference that prototype 3 comprises an extinguishing ring that is made of cork, which has the ability to extinguish the flame caused by the prototype. Therefore, when the flame reached the height of the cork ring, said flame was extinguished, without the need for additional intervention to extinguish said flame.

### Assessment of the results

To analyse the effectiveness of the 3 prototypes, the remains of the same were taken, and the strip of plant tissue that made up each of the three prototypes was unrolled.

In all three cases, it was possible to verify how the earwax plug, which had been extracted by the effect of the vacuum that was generated inside the tool, which is responsible for its suction force, was located on the internal face of the strip.

Lastly, it must be taken into account that the present invention must not be limited by the embodiment described herein. Other configurations may be carried out by those skilled in the art based on the present description. Accordingly, the scope of the invention is defined by the following claims.

## Claims

1. A tool (1) for removing earwax from the ear canal, **characterised in that**:
- it has a hollow cone shape and a lateral length from the vertex (1.1) to the base (1.2) thereof that is comprised between 16 to 25 cm, and
- it is made up of a plant tissue comprising between 70% to 100% hemp fibres, and wherein said plant tissue has a wax coating comprising at least 90% beeswax.

2. The tool according to claim 1, wherein the vertex (1.1) has a hole with a diameter between 4 mm and 10 mm.

3. The tool according to claim 1 or 2, wherein the base (1.2) has a hole with a diameter between 2 cm and 3.5 cm.

4. The tool according to any one of claims 1 to 3, wherein the plant tissue comprises between 1% and 30% of plant fibres that are selected from the group consisting of cotton, reed, palm, esparto, loofah, linen, jute fibres and a combination of the above.

5. The tool according to any one of claims 1 to 4, wherein the coating comprises between 1% and 10% of at least one wax-additive that is selected from the group consisting of carnauba wax, jojoba wax and a combination of the above.

6. The tool according to any one of claims 1 to 5, wherein the tool has a ring (2) that is perpendicular to the longitudinal axis of the tool and concentric with the holes of the vertex (1.1) and of the base (1.2) that is at least one quarter of the total lateral length of the tool away from the vertex (1.1).

7. The tool according to claim 6, wherein the ring (2) is a mark of a colour different from the colour of the wall of the tool (1).

8. The tool according to claim 6, wherein the ring (2) is made of a self-extinguishing material.

9. The tool according to any one of claims 6 to 8, wherein the ring has a thickness comprised between 5 to 10 mm.

10. A method for obtaining a tool (1) described in any one of claims 1 to 5, comprising the following steps:
- having a strip made up of a plant tissue comprising between 70% to 100% hemp fibres and that has a width of 1.5 to 6 cm and a length of 15 to 60 cm,
- soaking the strip with the wax coating comprising at least 90% beeswax and is at temperature comprised between 75 °C to 90 °C,
- cooling the strip soaked with the wax coating to a temperature comprised between 50 °C to 65 °C,
- wrapping the strip soaked with the wax coating around a cone-shaped mould following a helical movement from the vertex to the circular base of the mould, half the width of the strip to be placed on the mould overlapping the half of the strip that is arranged on the mould forming the funnel-shaped tool (1),
- cooling the tool (1) until it reaches a temperature comprised between 18 °C and 25 °C,
- demoulding the tool (1) and settling for at least 10 days at a temperature comprised between 18 °C and 25 °C.

11. The method for obtaining a tool (1) according to claim 10, wherein the strip is soaked with the coating on one face and during the step of shaping the tool (1), the face soaked with the coating is arranged towards the mould surface.

12. The method for obtaining a tool (1) according to claim 10 or 11, wherein the method, after the step of shaping the tool (1) or cooling the tool (1), comprises painting on the wall of the tool (1) a ring (2) that is perpendicular to the longitudinal axis of the tool and concentric with the holes of the vertex (1.1) and of the base (1.2) at least one quarter of the total lateral length of the tool (1) away from the vertex (1.1), said ring (2) being of a colour different from the wall of the tool (1).

13. The method for obtaining a tool (1) according to claim 10 or 11, wherein the method, after the step of shaping the tool (1) in a funnel shape and before cooling the tool (1), comprises bonding to the wall of the tool (1) a ring (2) that is perpendicular to the longitudinal axis of the tool and concentric with the holes of the vertex (1.1) and of the base (1.2) at least one quarter of the total lateral length of the tool away from the vertex (1.1), said ring (2) being made of a self-extinguishing material.

14. A use of the tool (1) defined in any one of claims 1 to 9 for removing earwax from the ear canal by means of the combustion of the tool (1).

15. The use according to claim 10, wherein the combustion of the tool (1) lasts for a time comprised between 8 to 15 minutes.
